(19) Europäisches Patentamt / European Patent Office / Office européen des brevets

(11) **EP 2 121 038 B1**

(12) **EUROPEAN PATENT SPECIFICATION**

(45) Date of publication and mention
of the grant of the patent:
**04.01.2017 Bulletin 2017/01**

(21) Application number: **08728269.5**

(22) Date of filing: **25.01.2008**

(51) Int Cl.:
*A61K 49/04* (2006.01)        *C01G 27/02* (2006.01)
*C01G 35/00* (2006.01)        *C09C 3/12* (2006.01)

(86) International application number:
**PCT/US2008/052007**

(87) International publication number:
**WO 2008/092059 (31.07.2008 Gazette 2008/31)**

(54) **TANTALUM OXIDE NANOPARTICLES AS IMAGING AGENTS FOR X-RAY/COMPUTED TOMOGRAPHY AND METHODS FOR MAKING SAME**

TANTALUMOXID-NANOPARTIKEL ALS ABBILDUNGSMITTEL FÜR RÖNTGEN-/COMPUTERTOMOGRAPHIE UND VERFAHREN ZU IHRER HERSTELLUNG

AGENTS DE FORMATION D'IMAGES À BASE DE NANOPARTICULES DESTINÉS À LA TOMOGRAPHIE À RAYONS X/À LA TOMODENSITOMÉTRIE ET PROCÉDÉS DE FABRICATION DE CEUX-CI

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.01.2007   US 627529**

(43) Date of publication of application:
**25.11.2009   Bulletin 2009/48**

(73) Proprietor: **General Electric Company**
**Schenectady, NY 12345 (US)**

(72) Inventors:
• **COLBORN, Robert Edgar**
**Niskayuna, New York 12309 (US)**
• **BALES, Brian**
**Niskayuna, New York 12309 (US)**
• **BONITATIBUS, JR., Peter**
**Saratoga Springs, New York 12866 (US)**
• **KULKARNI, Amit**
**Clifton Park, New York 12065 (US)**
• **TORRES, Andrew**
**Troy, New York 12180 (US)**
• **AXELSSON, Oskar**
**221-84 Lund (SE)**
• **DEMOULPIED, David**
**New Baltimore, New York 12124 (US)**
• **BUCKLEY, Paul**
**Scotia, New York 12302 (US)**

(74) Representative: **O'Brien, Dominic Paul et al**
**GE Healthcare Limited**
**Nightingales Lane**
**Chalfont St Giles**
**Buckinghamshire HP8 4SP (GB)**

(56) References cited:
WO-A-03/016217        WO-A-03/075961
WO-A-2005/051435      WO-A-2007/055995
US-A- 5 989 580

• CHAN D C ET AL: "Radiopacity of tantalum oxide nanoparticle filled resins." DENTAL MATERIALS : OFFICIAL PUBLICATION OF THE ACADEMY OF DENTAL MATERIALS MAY 1999, vol. 15, no. 3, May 1999 (1999-05), pages 219-222, XP002485048 ISSN: 0109-5641
• IWASAKI, KENTARO ET AL: "Preparation and Characterization of Water-Soluble Jingle-Bell-Shaped Silica-Coated Cadmium Sulfide Nanoparticles" JOURNAL OF PHYSICAL CHEMISTRY B , 108(32), 11946-11952 CODEN: JPCBFK; ISSN: 1520-6106, 2004, XP002485239
• WANG, CHUN ET AL: "Micropatterning of polystyrene nanoparticles and its bioapplications" COLLOIDS AND SURFACES, B: BIOINTERFACES , 46(4), 255-260 CODEN: CSBBEQ; ISSN: 0927-7765, 2005, XP005237689

- KANNAN, BALAJI ET AL: "Chemical patterning for the highly specific and programmed assembly of nanostructures" JOURNAL OF VACUUM SCIENCE & TECHNOLOGY, B: MICROELECTRONICS AND NANOMETER STRUCTURES--PROCESSING, MEASUREMENT, AND PHENOMENA , 23(4), 1364-1370 CODEN: JVSTBM; ISSN: 1071-1023, 2005, XP002485240
- PAPRA A ET AL: "Characterization of ultrathin poly(ethylene glycol) monolayers on silicon substrates" LANGMUIR AMERICAN CHEM. SOC USA, vol. 17, no. 5, 6 March 2001 (2001-03-06), pages 1457-1460, XP002485241 ISSN: 0743-7463
- AGRAWAL MUKESH ET AL: "Synthesis of novel tantalum oxide sub-micrometer hollow spheres with tailored shell thickness." LANGMUIR : THE ACS JOURNAL OF SURFACES AND COLLOIDS 5 FEB 2008, vol. 24, no. 3, 5 February 2008 (2008-02-05), pages 1013-1018, XP002485049 ISSN: 0743-7463
- ANTALEK, BRIAN: "Using PGSE NMR for chemical mixture analysis: quantitative aspects" CONCEPTS IN MAGNETIC RESONANCE, PART A: BRIDGING EDUCATION AND RESEARCH , 30A(5), 219-235 CODEN: CMRPC2; ISSN: 1546-6086, 2007, XP002485242
- Bert Braune ET AL: "Tantalum oxide nanomers for optical applications", Proc. SPIE 3469, Organic-Inorganic Hybrid Materials for Photonics, 6 July 1998 (1998-07-06), pages 124-132, XP055110581, Retrieved from the Internet: URL:http://scidok.sulb.uni-saarland.de/vol ltexte/2010/2894/ [retrieved on 2014-03-28]

**Description**

TECHNICAL FIELD

The present invention relates generally to imaging agents for use in X-ray/computed tomography, and more specifically to nanoparticle-based imaging agents and methods for making same.

BACKGROUND INFORMATION

Iodinated benzoic acid derivatives continue to serve as standard X-ray/computed tomography (CT) imaging agents, despite the risk factors and side effects associated with intravenous iodine injection. Additionally, such standard CT imaging agents are typically of low molecular weight, and they are known to clear from the human body very rapidly, making it difficult to target these agents to disease sites (Shi-Bao Yu and Alan D. Watson, Chem. Rev. 1999, 99, 2353-2377).

The literature describes experimental nanoparticle systems containing gadolinium (Gd) or iodine (I) for CT imaging. However, in such systems, only a relatively small number of heavy atoms may be delivered to/in the vicinity of the target tissues. Such approaches include a liposomal approach, in which iodinated molecules are encapsulated into liposomes (Leike et al., Invest. Radiol. 2001, 36(6), 303-308), as well as a dendritic approach, in which iodine atoms are conjugated to G-4 Starburst® polyamidoamide (PAMAM) dendrimers (Yordanov et al., Nano Letters 2002, 2(6), 595-599). Both approaches deliver, at most, a couple hundred heavy metal (i.e., gadolinium) atoms.

Efforts to deliver a greater number of heavy metal atoms have included the use of nanoparticles of such heavy metals. See PCT International Publication Nos. WO 03/075961 A2 and WO 2005/051435 A2. Although nanoparticles of elemental (zerovalent) metal species have the highest density (number of heavy metal atoms/volume), they suffer from issues such as robust synthesis and instability due to oxidation. Nanoparticles of inert metals such as gold (e.g., such as described in WO 03/075961 A2) can overcome these issues, but are not very cost effective.

Chan et al. (1999 Dental Materials; 15(3): 219-222) and Braune et al. (1998; Proc. SPIE 3469, Organic-Inorganic Hybrid Materials for Photonics: 124-132) disclose nanoparticles having a tantalum oxide core but these are for use in applications other than *in vivo* imaging where the nanoparticles are required to be hydrophobic.

WO 07/055995 A2 (Bonitatebus et.al.) describes core/shell nanoparticles wherein at least one of the nanoparticle core and the nanoparticle shell comprises an active contrast agent material.

Surface coatings for nanoparticles are known in the art. For example, Iwasaki et al. (2004 J Phys Chem; 108(32): 11946-11952) and WO03016217 disclose use of diethylphosphatoethyltriethoxysilane for coating and stabilizing nanoparticles. However these teachings are in the fields of photoetching and abrasion-resistant coatings, respectively. PEG-silanes are known for coating surfaces e.g. as taught by Wang et al. (2005 Colloids and Surfaces, B: Biointerfaces; 46(4): 255-260), Kannan et al. (2005 J Vacuum Sci Technol B: Microelectronics and Nanometer Structures - Processing, Measurement and Phenomena; 23(4): 1364-1370) and Papra et al. (2001 Langmuir Am Chem Soc USA; 17(5): 1457-1460). However these disclosures are not concerned with *in vivo* imaging applications.

As a result of the foregoing, there is a continuing need for new imaging agents for CT, especially to the extent that such imaging agents can provide for improved performance and benefit in one or more of the following areas: robust synthesis, reduced cost, image contrast enhancement, increased blood half-life, decreased toxicity, decreased radiation dose, and targeting capability.

BRIEF DESCRIPTION OF THE INVENTION

The present invention is generally directed to core/shell nanoparticles, wherein such core/shell nanoparticles comprise a nanoparticle core and a nanoshell disposed about the nanoparticle core such that, in the aggregate, they form a core/shell nanoparticle that is operable for use as an imaging agent in X-ray imaging, particularly computed tomography (CT) imaging.

The present invention is directed to an imaging agent comprising an active nanoparticle core comprising tantalum (Ta) in a non-zero valent state, and a passive nanoshell, the nanoshell being disposed about the nanoparticle core such that in the aggregate they form a core/shell nanoparticle that is operable for use as an imaging agent in CT imaging.

In some embodiments, an X-ray/computed tomography imaging solution comprises an ensemble of the imaging agents according to any of the embodiments described herein, wherein the mean diameter of the ensemble is not more than about 10 nm, preferably not more than about 3 nm.

In some embodiments, the present invention is directed to methods of making any of the above-described imaging agents.

In some embodiments, the present invention is directed to a method for making an X-ray/computed tomography imaging agent, the method comprising the steps of: providing a first precursor material comprising tantalum; forming an active core from the first precursor material, the core comprising tantalum in a non-zero valent state; providing a second precursor material; and forming a passive shell from the second precursor material, wherein the passive shell is disposed about the core such that the core and the shell form a core/shell nanoparticle.

The CT imaging agent of the present invention delivers a relatively large number of high-density, highly-attenuating tantalum atoms in molecular form to improve CT contrast enhancement. The CT imaging agent can be used for targeting of specific disease sites or for macrophage uptake, or can have increased blood half-life.

The foregoing has outlined rather broadly the features of the present invention in order that the detailed description of the invention that follows may be better understood. Additional features and advantages of the invention will be described hereinafter which form the subject of the claims of the invention.

BRIEF DESCRIPTION OF THE DRAWINGS

For a more complete understanding of the present invention, and the advantages thereof, reference is now made to the following descriptions taken in conjunction with the accompanying drawings, in which:

FIGURE 1 generally depicts a cross-sectional view of a core/shell nanoparticle so as to illustrate the relationship between the components;

FIGURE 2 depicts a cross-sectional view of a core/shell nanoparticle comprising an active core and a passive shell, in accordance with the present invention;

FIGURE 3 illustrates, in flow diagram form, a method of using core/shell nanoparticles as imaging agents for computed tomography;

FIGURE 4 is a plot of the distribution of diameter of an ensemble of active core/passive shell nanoparticles comprising a tantalum oxide core and a polyethylene glycol (PEG) polymeric shell, in accordance with some embodiments of the present invention;

FIGURE 5 is a TEM image of active core/passive shell nanoparticles with tantalum oxide and a PEG polymeric shell, in accordance with some embodiments of the present invention;

FIGURE 6 is a plot of the distribution of diameter of an ensemble of active core/passive shell nanoparticles comprising a tantalum oxide core and a citrate ligand shell, in accordance with some embodiments of the present invention;

FIGURE 7 is a TEM image of active core/passive shell nanoparticles comprising a tantalum oxide core and a citrate ligand shell, in accordance with some embodiments of the present invention;

FIGURE 8 is a plot of the percentage of viable cells versus molar amount of tantalum for active core/passive shell nanoparticles with a tantalum oxide core and a PEG polymeric shell and for active core/passive shell nanoparticles with a tantalum oxide core and a citrate ligand shell, in accordance with some embodiments of the present invention;

FIGURE 9 is a plot of a C3a standard curve to detect C3a in solution in accordance with some embodiments of the present invention;

FIGURE 10 is a bar chart of C3a for active core/passive shell nanoparticles with a tantalum oxide core and each of the following shells: a glucose shell, an EDTA shell, and PEG shell in accordance with some embodiments of the present invention, and for various controls.

FIGURE 11 is a computed tomography image of a rat injected with active core/passive shell nanoparticles with a tantalum oxide core and a PEG shell, in accordance with some embodiments of the present invention;

FIGURE 12 is a series of computed tomography images at different times of a rat injected with active core/passive shell nanoparticles with a tantalum oxide core and a PEG shell, in accordance with some embodiments of the present invention.

DETAILED DESCRIPTION OF THE INVENTION

The present invention is generally directed to core/shell nanoparticles, wherein such core/shell nanoparticles comprise a nanoparticle core and a nanoshell disposed about the nanoparticle core such that, in the aggregate, they form a core/shell nanoparticle that is operable for use as an X-ray imaging agent, particularly in CT.

Referring to the drawings in general, it will be understood that the illustrations are for the purpose of describing a particular embodiment of the invention and are not intended to limit the invention thereto.

While most of the terms used herein will be recognizable to those of skill in the art, the following definitions are nevertheless put forth to aid in the understanding of the present invention. It should be understood, however, that when not explicitly defined, terms should be interpreted as adopting a meaning presently accepted by those of skill in the art.

"Computed Tomography," abbreviated "CT," as defined herein and also known as computed axial tomography or computer-assisted tomography (CAT) and body section roentgenography, is a medical imaging method employing tomography where digital processing is used to generate a three-dimensional image of the internals of an object (or subject) from a large series of two-dimensional X-ray images taken around a single axis of rotation. While the discussion herein focuses on computed tomography, it will be appreciated by those of skill in the art that such discussions apply generally to all types of X-ray imaging.

"Imaging agents," as defined herein and also known as contrast agents, are agents that comprise a material that can significantly attenuate incident X-ray radiation causing a reduction of the radiation transmitted through the volume of interest. After undergoing CT image reconstruction and typical post-processing, this increased X-ray attenuation is interpreted as an increase in the density of the volume of interest, which creates a contrast enhancement in the volume comprising the contrast agent relative to the background tissue in the image. Because the discussion herein is generally applicable to all forms of X-ray imaging, the imaging agents of the present invention are generally referred to herein as "X-ray/computed tomography imaging agents." This term is used interchangeably with "computed tomography (CT) agents."

In reference to the core and shell components of a core/shell nanoparticle, the terms "active" and "passive" refer to the components' ability to create a contrast enhancement in CT imaging. A conventional CT scanner scans uses a broad spectrum of X-ray energy between about 10 keV and about 140 keV. Those skilled in the art will recognize that the amount of X-ray attenuation passing through of a particular material per unit length is expressed as the linear attenuation coefficient. At an X-ray energy spectrum typical in CT imaging, the attenuation of materials is dominated by the photoelectric absorption effect and the Compton Scattering effect. Furthermore, the linear attenuation coefficient is well known to be a function of the energy of the incident X-ray, the density of the material (related to molar concentration), and the atomic number (Z) of the material. For molecular compounds or mixtures of different atoms the 'effective atomic number,' $Z_{eff}$, can be calculated as a function of the atomic number of the constituent elements. The effective atomic number of a compound of known chemical formula is determined from the relationship:

$$Z_{eff} = \left[ \sum_{k=1}^{P} w_{f_k} Z_k^{\beta} \right]^{1/\beta} \qquad \text{(Eq. 1)}$$

where $Z_k$ is the atomic number of simple elements, $P$ is the total quantity of simple elements, and $w_{f_k}$ is the weight fraction of simple elements with respect to the total molecular weight of the molecule (related to the molar concentration). The optimal choice of the incident X-ray energy for CT imaging is a function of the size of the object to be imaged and is not expected to vary much from the nominal values. It is also well known that the linear attenuation coefficient of the contrast agent material is linearly dependent on the density of the material, i.e., the linear attenuation coefficient can be increased if the material density is increased or if the molar concentration of the contrast material is increased. However, the practical aspects of injecting contrast agent material into patients, and the associated toxicity effects, limit the molar concentration that can be achieved. Therefore, it is reasonable to separate potential contrast agent materials according to their effective atomic number. Based on simulations of the CT contrast enhancement of typical materials for a typical CT energy spectrum with a molar concentration of approximately 50 mM, it is estimated that materials with effective atomic number greater than or equal to 34 will yield appropriate contrast enhancement of about 30 Hounsfield units (HU), or 3% higher contrast than water. Therefore, we have defined potential CT contrast materials as being either passive, having an effective atomic number of less than 34 (i.e., $Z_{eff} < 34$), or active, having an effective atomic number greater than or equal to 34 (i.e., $Z_{eff} \geq 34$). See, e.g., Chapter 1 in Handbook of Medical Imaging, Volume 1, Physics and Psychophysics, Eds. J. Beutel, H.L. Kundel, R.L. Van Metter, SPIE Press, 2000).

A "nanoparticle," as defined herein, is a particle with an average diameter of between about 1 nm and about 500 nm, and can be used to refer to nanoparticle cores and core/shell nanoparticle aggregates. Such nanoparticles can be spherical or irregularly shaped, and, particularly on the smaller end of this size range, are differentiated from molecular complexes. A "nanoshell," as defined herein, is the shell region of the core/shell nanoparticle. Such a nanoshell is disposed about the nanoparticle core in such a way that it may or may not conform to the topography of the underlying nanoparticle core.

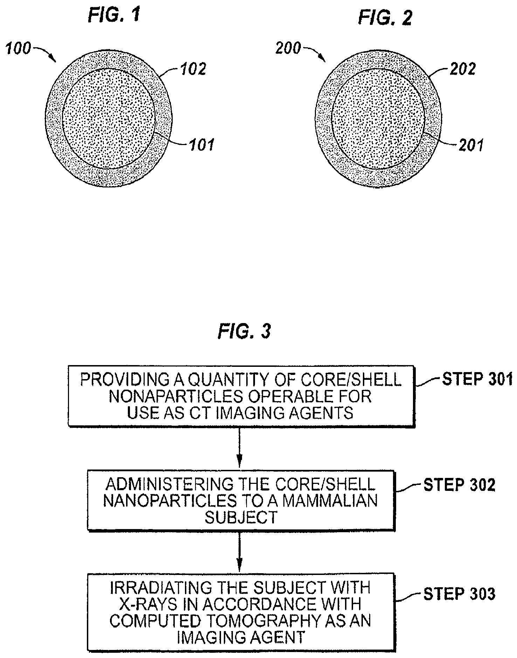

As mentioned above, and referring to FIGURE 1, in some embodiments the present invention is directed toward an imaging agent comprising: (a) a nanoparticle core 101; and (b) a nanoshell 102, the nanoshell being disposed about the nanoparticle core such that, in the aggregate, they form a core/shell nanoparticle 100 that is operable for use as an imaging agent in computed tomography (generally, X-ray) imaging. While FIGURE 1 depicts a cross-section of a perfectly spherical core/shell nanoparticle, such core/shell nanoparticles can also be irregularly-shaped.

The above-described imaging agent can further comprise at least one targeting agent. Such targeting agents are useful for targeting the imaging agents to specific diseased regions of a subject's body. Typically, the targeting agent is an antibody (e.g., IgG) or other peptide, but can also be a nucleic acid (e.g., DNA, RNA) or other suitable chemical species. Generally, the targeting agent is attached to one or both of the nanoparticle core and the nanoshell disposed about the nanoparticle core. Such attachment typically comprises a linkage such as, but not limited to, a peptide linkage, a disulfide linkage, an isothiourea linkage, an isourea linkage, a sulfonamide linkage, an amine linkage, a carbamate linkage, an amidine linkage, a phosphoramidate linkage, a thioether linkage, an arylamine linkage, an aryl thioether linkage, an ether linkage, a hydrazone linkage, a traizole linkage, an oxime linkage, and combinations thereof. See, e.g., Chapter 2 in Bioconjugate Techniques. G. T. Hermanson, Academic Press, 1996.

In some embodiments, the above-described CT imaging agents are optimized for macrophage uptake via control of the core/shell nanoparticle surface charge and/or the presentation of functional groups which induce macrophage uptake (e.g., polyvinyl sulfate).

For the core/shell nanoparticles described herein for use as imaging/contrast agents in CT, the nanoparticle core typically has an average diameter of from about 1 nm to about 100 nm, more typically from about 2 nm to about 80 nm, and most typically from about 2 nm to about 20 nm. The nanoshell disposed about the nanoparticle core typically has an average thickness of from about 0.5 nm to about 100 nm. Accordingly, the aggregate of the nanoparticle core and the nanoshell of the imaging agent typically has an average diameter of from about 2 nm to about 500 nm, more typically from about 2 nm to about 100 nm, and most typically from about 3 nm to about 20 nm.

It will be understood that it may be desirable for the aggregate of the nanoparticle core and the nanoshell to be excretable by a mammalian kidney, typically a human kidney. Accordingly, the aggregate of the nanoparticle core and the nanoshell of the imaging agent may have an average diameter of from about 1 to about 20 nm, more typically from about 2 to about 12 nm, and most typically from about 3 to about 8 nm.

In some embodiments, an X-ray/computed tomography imaging solution comprises an ensemble of the imaging agents according to any of the embodiments described herein, wherein the mean diameter of the ensemble is not more than about 10 nm, more typically not more than about 7 nm, more typically not more than about 6 nm and most typically not more than 3 nm.

Active Core/Passive Shell Nanoparticles

Referring to FIGURE 2, the present invention is directed to an imaging agent 200 comprising an active nanoparticle core 201 and a passive nanoshell 202, the nanoshell being disposed about the nanoparticle core such that in the aggregate they form a core/shell nanoparticle 200 that is operable for use as an imaging agent in CT imaging.

The material of which the above-described nanoparticle core 201 is comprised is limited in that it comprises an active material for contrast enhancement in CT ($Z_{eff} \geq 34$), where the active material comprises tantalum in a non-zero valent state. The nanoparticle core typically comprises material such as, but not limited to, tantalum oxides. By way of example and not limitation, the effective atomic number, $Z_{eff}$, for $Ta_2O_5$ is about 69.

The material of which the nanoshell 202 is comprised is not particularly limited, but generally does not comprise active CT contrast agent material ($Z_{eff} < 34$) and must generally be capable of being disposed about the nanoparticle core. Suitable such materials include, but are not limited to, ligands, oligomers, polymers, clusters, carbohydrate species, functionalized silica, and combinations thereof. For example, suitable shell materials include, but are not limited to, polyethylene glycol, polyethylene imine, polymethacrylate, polyvinylsulfate, polyvinylpyrrolidinone, citrate, malate, glycolate, silanes, and combinations thereof.

The material of which the nanoshell is made is water soluble and biocompatible.

The nanoshell preferably comprises diethylphosphatoethyltriethoxysilane (PHS) or 2-[Methoxy(poly-ethylenoxy)propyl]trimethoxysilane (PEGsilane550).

Methods of Making

In some embodiments, the present invention is directed to method of making any or all of the above-described types of imaging agents in CT applications.

Such methods may comprise the steps of: (a) providing a first precursor material comprising tantalum; (b) forming an active core from the first precursor material, the active core comprising tantalum in a non-zero valent state; (c) providing a second precursor material; and (d) forming a passive shell from the second precursor material, wherein the passive shell is disposed about the core such that the core and the shell form a core/shell nanoparticle. The passive shell comprises a water soluble material derived from the second precursor material. Alternatively, or in combination, in some embodiments, the method further comprises controlling the average diameter of the nanoparticle.

It will be understood that the order and/or combination of steps may be varied. Thus, according to some embodiments, steps (a), (b), (c), and (d) occur as sequential steps so as to form the nanoparticle from the active core and the second precursor. By way of example and not limitation, in some embodiments, the first precursor comprises tantalum in a non-zero valent state; wherein the core comprises an oxide of the tantalum in the same non-zero valent state; and wherein step (b) comprises hydrolysis of the first precursor. According to some embodiments, the first precursor is a salt, such as an alkoxide or halide, of tantalum, and the hydrolysis proceeds upon combining the first precursor, an acid, water, or water analog such as deuterium oxide, in alcoholic solvent. The water, or water analog, initiates the hydrolysis. The molar ratio of the acid to tantalum may be selected so as to control the size of the nanoparticle core. According to some embodiments, when the core comprises an oxide of tantalum, a silicon-containing material is attached to the core via Si-O linkages. According to some embodiments, the siloxane includes, but is not limited to polymerizable groups. The polymerization may proceed via acid induced condensation polymerization. Alternatively, according to some embodiments, a polymer may be physi-sorbed on the core. According to any of the above-described embodiments, the polymer is water soluble and biocompatible. Suitable polymers are polyethylene glycol (PEG), polyethylene imine (PEI), polyvinylsulfate and polyvinylpyrrolidinone, and combinations thereof.

Alternatively, according to some embodiments, steps (a) and (c) occur together such that steps (b) and (d) occur together so as to form the nanoparticle directly from the first and second precursors. By way of example and not limitation, according to some embodiments, the first precursor comprises tantalum in a zero valent state; wherein the core comprises an oxide of tantalum; and wherein steps (b) and (d) together comprise adding the first and second precursors to a basic aqueous solution. According to some embodiments, the basic solution has a pH of at least about 9. Direct formation of the core and shell in the same step, such as by combining steps (a) and (c) is an unexpected discovery. Further, we have surprisingly discovered that exchange reactions which are well known for metal nanoparticles, such as those involving citrate shell formation on gold nanoparticles, may be used for making ligand based shells disposed about non-metallic cores, using, for example, the present direct preparation. When the shell comprises a ligand, the first precursor may comprise a ligand reagent. For example, when the ligand is a carboxylic acid anion, such as citrate, the ligand reagent is the associated carboxylic acid. Suitable alternative ligand reagents include, but are not limited to, sugars, alcohols, polyaldehydes, and combinations thereof. Glucose is an example that allows for the preparation of very small nanoparticles. Other suitable reagents include, but are not limited to, glycolic acid and malic acid. According to some embodiments, the diameter of the nanoparticles is controlled via selection of the identity and/or amount of ligand.

In combination with any of the above-described embodiments, in some embodiments, a method of making a X-ray/computed tomography preparation (e.g. containing a plurality of imaging agents) comprises forming an ensemble of core/shell nanoparticles, wherein the mean diameter of the ensemble is not more than about 10 nm, more typically not more than about 7 nm, most typically not more than about 6nm; in some embodiments the mean diameter is not more than about 3 nm. It will be understood that according to some embodiments, the mean diameter is selected so as to render the nanoparticles in the ensemble substantially clearable by a mammalian kidney, such as a human kidney, in particulate form.

The mean diameter of the ensemble can be controlled using any technique suitable for controlling the size distribution of nanoparticles in solution. Accordingly, in some embodiments, forming the ensemble described above includes a fractionation step, wherein a raw ensemble in solution is passed through a process that divides the raw ensemble into at least two populations having different particle size. Several suitable fractionation techniques are known in the art, including, for instance, various filtration processes. Normal filtration, where a particle-containing fluid is forced directly toward a filter membrane under an applied pressure (as created by, for example, centrifugal force or other means), is a common technique. Another suitable technique is tangential flow filtration, where the fluid is pumped tangentially along the surface of the membrane with an applied pressure that serves to force a portion of the fluid through the membrane. In this technique, unlike in normal filtration, the retained, larger particles are swept along by the tangential flow, rather than accumulating at the surface of the membrane. This sweeping effect is often advantageous in achieving efficient size-based separation of very fine particulate ensembles. Another example of a suitable technique is diafiltration, a type of tangential flow filtration process in which buffer is added to the process stream as filtrate is removed. One skilled in the art is familiar with these processes and how to use them to obtain a desired size distribution for a nanoparticle ensemble. In some embodiments, the raw nanoparticle ensemble is fractionated to provide a product ensemble of nanoparticles having mean diameter in accordance with any of the ranges given above.

In some embodiments, the X-ray/computed tomography preparation is purified to remove undesirable species that may interfere with or otherwise degrade the performance of the preparation. Techniques such as dialysis are suitable for this purpose. Moreover, techniques such as diafiltration have been shown to effectively purify and fractionate nanoparticle solutions in one processing step. See, for example, Sweeney et al., J. Am. Chem. Soc. 2006, vol.128, pp 3190-3197.

Methods of Using

The above-described types of imaging agents can be used in CT applications. Referring to FIGURE 3, such methods typically comprise the steps of: (Step 301) providing a quantity of core/shell nanoparticles comprising an active computed tomography contrast agent material, the core/shell nanoparticles each comprising a nanoparticle core and a nanoshell disposed about the nanoparticle core; (Step 302) administering the core/shell nanoparticles to a mammalian subject; and (Step 303) irradiating the subject with X-rays, in accordance with computed tomography, such that the core/shell nanoparticles serve as an imaging agent. Such core/shell nanoparticles may further comprise targeting agents such that, as imaging agents, they can be targeted to specific diseased areas of the subject's body. In some embodiments, the above-described core/shell nanoparticles, to the extent that they persist in the blood, are blood pool agents.

In some such above-described methods of using the above-described imaging agents, the core/shell nanoparticles provide for a CT signal of generally at least about 5 Hounsfield units to at most about 5000 Hounsfield units, and more particularly at least about 100 Hounsfield units to at most about 5000 Hounsfield units.

The following examples are included to demonstrate particular embodiments of the present invention.

EXAMPLE 1

This Example serves to illustrate how a CT imaging agent can be prepared, in accordance with some embodiments of the present invention. In this particular Example, a polymeric passive shell is linked to an active core of tantalum oxide. Further, in this particular Example, the shell is formed about the core after the core is formed.

This example illustrates preparation of $Ta_2O_5$ nanoparticles for X-ray imaging: 34 ml of *n*-propanol, 0.44 ml isobutyric acid and 0.5 ml deuterium oxide were combined under nitrogen in the order specified and stirred for 30 minutes at room temperature. Tantalum ethoxide (1.87 g) was added in a drop-wise manner, albeit rapidly, and stirring continued under nitrogen for 18 hours. The tantalum ethoxide contains Ta(V), that is tantalum in the +5 valence state, a non-zero valence state. 2-[Methoxy(poly-ethylenoxy)propyl]trimethoxysilane (PEGsilane550, 4.832 g) was added to the stirred mixture as a 40 ml solution in *n*-propanol and the reaction was refluxed for 1 hour in air. Once cooled to room temperature, HCl was added (125 $\mu$l, 0.1 M) and the reaction was allowed to stir overnight. Deionized water (40 ml) was added, the mixture stirred, and then all volatiles were removed to obtain a clear, colorless-to-slightly yellow gel-like product. To purify the nanoparticles for intravenous injection, the product was solubilized in deionized water and filtered through a 100 nm membrane. Dialysis in water for 12 hours (3500-8000 MWCO dialysis tubing), followed by 100 nm filtration and subsequent removal of water via lyophilization, yielded an off-white precipitate approximately 22 % wt/wt in tantalum. Solutions as high as 1.8 M in tantalum have been prepared using saline (0.9 % NaCl).

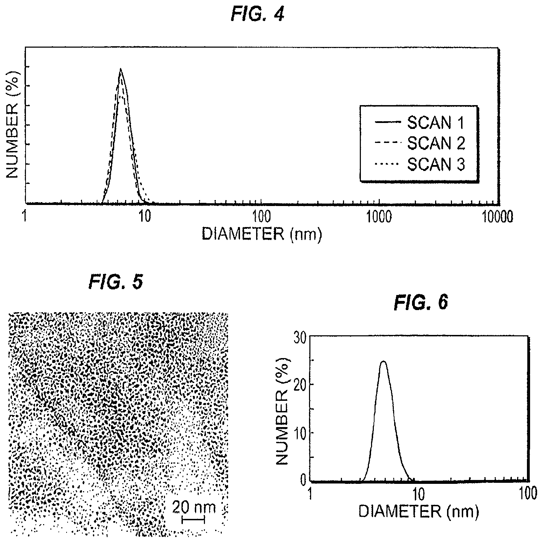

FIGURE 4 shows the distribution of diameters for an ensemble of the nanoparticles made according to this Example, as measured by conventional DLS, demonstrating a mean diameter of the ensemble of 7 nm. FIGURE 5 shows a TEM image of nanoparticles made according to this Example.

EXAMPLE 2

This Example serves to illustrate how a CT imaging agent can be prepared, in accordance with some embodiments of the present invention. In this particular Example, a polymeric passive shell is linked to an active core of tantalum oxide. Further, in this particular Example, the shell formed about the core after the core is formed.

This example illustrates preparation of $Ta_2O_5$ nanoparticles for X-ray imaging: 34 ml of *n*-propanol, 0.44 ml isobutyric acid, and 0.5 ml deuterium oxide were combined under nitrogen in the order specified and stirred for 30 minutes at room temperature. Tantalum ethoxide (1.87 g) was added in a drop-wise manner, albeit rapidly, and stirring continued under nitrogen for 18 hours. The tantalum ethoxide contains Ta(V), that is tantalum in the +5 valence state, a non-zero valence state. Next, diethylphosphatoethyltriethoxysilane (PHS, 3 g) was added to the mixture as a 40 ml solution in *n*-propanol and the reaction was refluxed for 1.5 hours in air. Once cooled to room temperature, ammonium hydroxide was added (250 $\mu$l, 0.1 M) and the reaction was allowed to stir overnight. Deionized water (40 ml) was then added to the reaction with stirring, and then hydrochloric acid was added (10 ml, 1.2 M) and allowed to react for 2 days at 50°C. Upon cooling, the reaction was neutralized with ammonium hydroxide to pH ~ 7-8 and filtered through a 100 nm membrane. All volatiles were removed to give the product. To purify the nanoparticles for intravenous injection, the product was solubilized in deionized water (pH 7.5-8), filtered through a 100 nm membrane, dialyzed in water for 12 hours (3500-8000 MWCO dialysis tubing), and lyophilized to yield a snow-white precipitate approximately 30 % wt/wt in tantalum. IR (nujol mull, NaCl plates, cm$^{-1}$): 1454 (very strong), 1413 (weak), 1376 (strong), 1296 (weak), 1274 (weak), 1218 (strong), 1170 (medium), 1029 (very strong), 964 (very strong), 782 (medium). NMR (D$_2$O, ppm): $^{31}$P, 37.4 (broad); $^1$H (broadened resonances), 4.16, 1.88, 1.37, 0.85.

## EXAMPLE 3

This Example serves to illustrate how a CT imaging agent can be prepared, in accordance with some embodiments of the present invention. In this particular Example, a citrate ligand passive shell is linked to an active core of tantalum oxide. Further, in this particular Example, the shell formed in conjunction with the core.

This example describes direct preparation of $Ta_2O_5$ nanoparticles from tantalum powder (325 mesh, Aldrich). The tantalum powder contained Ta(0), that is, tantalum in a zero valence state. A solution was prepared by combination of 20ml of 30% hydrogen peroxide and 5ml of ammonium hydroxide solution (28-30% $NH_3$ in water). These were combined in a 2-neck flask under nitrogen and cooled with an ice-bath. Subsequently, the addition of Ta powder (5mmol, 0.907g) was made and the mixture was allowed to stir for 2h (magnetic stirring may be used at small scale, but mechanical stirrer is more appropriate for larger scales). Then citric acid (15mmol, 3.152g) was added and a condenser was fitted to the flask. The slurry was heated to 80°C for 18h. The reaction was then allowed to cool and the remaining tantalum powder was filtered off through a medium grade glass frit. The filtrate was concentrated by removal of water through rotoevaporation. The remaining colored solution was then dialyzed with a 3.5K cutoff in an aqueous solution buffered to pH of 7.2. The resulting solution was analyzed for the nanoparticles. If desired, the nanoparticles may be isolated by lyophilization.

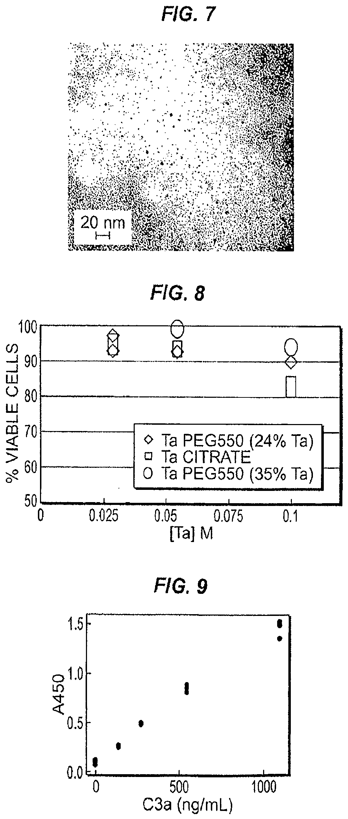

FIGURE 6 shows the distribution of diameters for an ensemble of the nanoparticles made according to this Example as measured by conventional DLS, demonstrating a mean diameter of the ensemble of 7 nm. FIGURE 7 shows a TEM image of nanoparticles made according to this Example.

## EXAMPLE 4

This Example serves to illustrate how a CT imaging agent can be used. In this particular Example, cell viability studies of active core/passive shell nanoparticles demonstrate no appreciable loss of cell viability over the studied time period.

THP1 monocytes (ATCC TIB-71) were diluted to final density of 1 x $10^6$ cells/ mL in RPMI 1640 +10% FBS media and 500uL of cells were aliquoted into each well of a 24 well plate. Sterile-filtered tantalum oxide nanoparticles (100mM Ta) were pipetted into wells. For control wells, either 50 uL of sterile 0.9% saline (negative control) or 80 uL of 30% hydrogen peroxide (positive control) was added to cells. The plate was swirled gently after addition and cells were cultured under normal conditions (37 °C, 5% carbon dioxide, 100% humidity).

Following incubation, cells were pipetted into centrifuge tubes and cells were centrifuged at 300 x g for 5 minutes at 20 degrees C, and the media was gently aspirated off. The cells were washed with 500 uL of 1X PBS, gently vortexed and centrifuged at 300 x g for 5 minutes at 20 °C. The cells were washed and the buffer was gently aspirated off. Annexin V FLUOS assay buffer (containing incubation buffer, Annexin V- fluorescein and propidium iodide) was prepared according to manufacturer's protocol (Annexin V FLUOS kit, Roche) and added 100uL to each tube. Tubes with cells were gently vortexed and incubated at room temperature for 15 minutes. An additional 300uL of incubation buffer were added to each tube prior to flow cytometry analysis.

The following were measured: the number of cells that were positive for Annexin V-fluorescein alone (apoptotic cclls), positive for Annexin V-fluorescein and propidium iodide (necrotic cells) or negative for both (viable cells).

Referring to FIGURE 8, all tantalum oxide preparations up to 0.1 mM Ta show negligible number of apoptotic or necrotic cells indicating no loss of cell viability after 3-hour exposure to nanoparticles.

## EXAMPLE 5

This Example serves to illustrate how a CT imaging agent can be used. In this particular Example, complement activation studies of active core/passive shell nanoparticles demonstrate no appreciable increase in C3a compared to untreated or saline-treated serums.

Human plasma and serum was purchased from a commercial vendor (Bioreclamation). To isolate serum, the whole blood was drawn into serum tubes and allowed to clot at room temperature for 30-45 minutes. The sample was centrifuged at 2000 x g for 10 minutes and the serum was immediately frozen at -70 °C. Samples were shipped overnight on dry ice and stored at -70 °C.

Serum was thawed at 37 °C for a few minutes in a water bath. 250 uL of serum was added into individual wells of a 24-well plate. Sterile test samples (Ta agent, saline and cobra venom factor) were added to sera to a final volume of 300uL. The 24-well plate was transferred to an incubator (37 °C) for 30 minutes with occasional agitation.

Referring to FIGURE 9, following the manufacturer's protocols (C3a assay, Quidel), stock solutions and dilutions were prepared to generate a C3a standard curve and high/low C3a samples.

To stop C3a production after 30 minute incubation, 37.5mM sodium citrate was added to each well and 0.5uL of serum (with and without test samples) was transferred to 1.5mL of kit sample buffer in Eppendorf tubes. The tubes were inverted 3 times gently to mix the serum and sample buffer, then 100 uL of the diluted serum was added to individual wells of the C3a test strips and incubated at room temperature for 60 minutes. The wells were washed three times with wash buffer (200 uL of wash buffer per wash, wash buffer in the well for 1 minute per wash). 100uL of C3a conjugate solution was added and the resulting combination incubated for 60 minutes at room temperature. The wash protocol was repeated (three washes, 200 uL of wash buffer per wash, wash buffer in the well for 1 minute per wash) then 100 uL of substrate was added to each well. After incubating 15 minutes, 100 uL of stop solution was added and the absorbance was measured at 450nm on a 96 well UV plate reader. C3a levels were calculated from C3a standard curve.

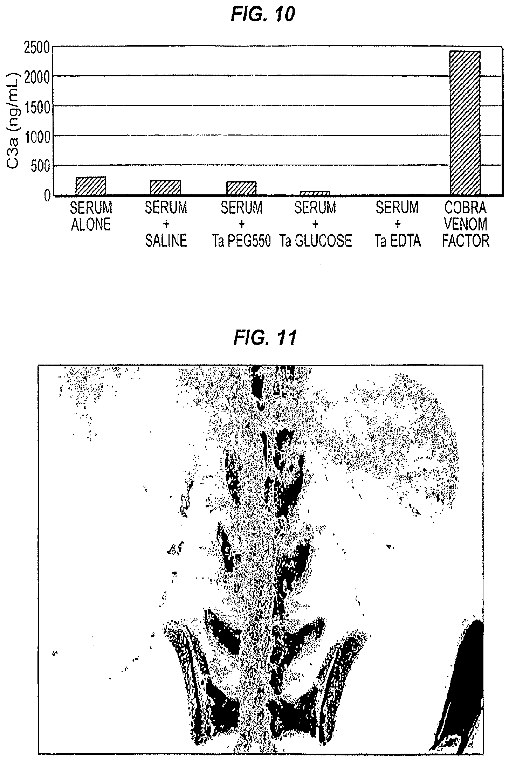

FIGURE 10 shows data from analysis. In particular, FIGURE 10 shows run data from incubation of tantalum oxide cores with each of the following shells; PEG, EDTA, citrate, and glucose in serum. Though some compounds (Ta EDTA) lowered C3a levels, none showed an increase in C3a compared to either untreated serum or saline-treated serum. No significant increase in C3a levels is observed by addition of up to 100 mM Ta. Elemental analysis via ICP-MS was used to determine total Ta content.

EXAMPLE 6

This Example serves to further illustrate how a core/shell nanoparticle-based CT imaging agent can be used to enhance contrast.

All imaging studies were performed on a EXplore Locus microCT imaging system using the following parameters: 80 kV, 4 minute acquisition.

A female Dark Agouti rat approximately 160g in body weight was anesthetized by IP injection of ketamine/diazepam. An IV catheter (24 gage) was placed in the tail vein and flushed with 200uL of sterile saline. The rat was then imaged on microCT from base of rib cage to bladder (Pre-injection scan). Immediately after the scan, the rat was injected via catheter with 1mL of 210mg Ta/mL tantalum oxide-PEG550 agent and scanned following completion of previous scan.

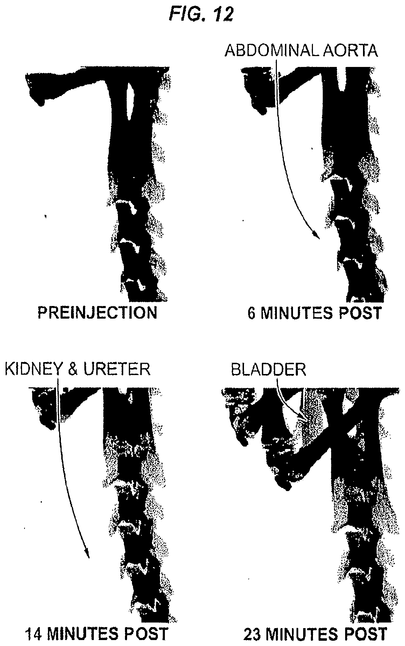

Referring to FIGURE 12, images were reconstructed at half resolution and viewed using maximum intensity projections. Immediately following injection, contrast was observed in abdominal aorta, vena cava and renal arteries. Within 14 minutes after injection, the agent was visible in kidney and ureters as it was excreted to bladder. Bladder contrast was prominent within 23 minutes after injection. Still referring to FIGURE 12, CT imaging studies show blood clearance between 6-14 minutes, followed by renal excretion to the bladder within 30 minutes.

The rat is imaged for 60 minutes after injection then returned to cage. After 24 hours, the rat is imaged again and no remaining contrast was observed in bladder or kidneys. No adverse effects are observed in injected rats.

FIGURE 11 shows an abdominal artery image that was obtained by microCT imaging using nanoparticles with $Ta_2O_5$ and PEG shells.

EXAMPLE 7

This Example serves to further illustrate how the mean diameter of an ensemble of core shell nanoparticles can be controlled by filtration, in accordance with some embodiments of the present invention. Further in this example solutions of different particle size was administered into laboratory rat subjects.

Tangential-Flow Filtration (TFF) was employed as a method of removing large tantalum oxide particles, in lieu of using 100nm or 20nm membrane filters in Normal Filtration (NF) mode. TFF-compatible molecular weight cut-off (MWCO) filters ranging between 100kD and 10kD were used in the TFF process for the purpose of removing larger nanoparticles. Permeates of filters in the specified range (100kD to 10kD MWCO) contained smaller tantalum oxide nanoparticles.

TFF was also employed as a method of purifying smaller tantalum oxide nanoparticles in said permeates, by removing low molecular-weight impurities, salts etc. in lieu of dialysis. Molecular-weight cut-off (MWCO) filters ranging between 10kD and 5kD were used in the TFF process for the purpose of removing impurities. Retentates of filters in the specified range (10kD to 5kD MWCO) contained purified small tantalum oxide nanoparticles.

Moreover, TFF was employed as a process to size-fractionate and purify tantalum oxide nanoparticles in sequential steps. For example, polydisperse tantalum oxide nanoparticles were processed by TFF using a 30kD MWCO filter thereby retaining ~6nm particles (retentate) and allowing passage of ~3nm particles (permeate). Permeates containing ~3nm particles, then served as retentates in a TFF process employing 10kD or 5kD MWCO filters.

TFF is an efficient and scalable process to produce nanoparticles of selected sizes, with a high degree of purity, than achievable by a combination of normal (membrane or centrifugal) filtration and dialysis techniques.

Three preparations of nanoparticle solutions, each having a different nominal mean particle size (20nm, 6nm, and 3nm), were prepared according to the methods described above and administered into laboratory rat subjects at identical dose levels. After a given amount of time, the kidneys of the rats were analyzed to determine concentrations of retained particles (calculated as mgTa/g kidney-sample). The results indicated that particle clearance was a strong function of mean particle size. For the kidney, the 6 nm particle concentration was lower than the 20 nm particle concentration by about 20%, and the 3nm particle concentration was lower than the 20 nm concentration by about 50%.

**Claims**

1. An X-ray/computed tomography imaging agent comprising:

    a) an active nanoparticle core, wherein the active nanoparticle core comprises tantalum (Ta) in a non-zero valent state;
    b) a passive nanoshell wherein the material of which the nanoshell is made is water soluble and biocompatible, and
    (c) the nanoshell is disposed about the nanoparticle core such that, in the aggregate, the active nanoparticle core and the passive nanoshell form a core/shell nanoparticle that is operable for use as an imaging agent in X-ray/computed tomography imaging.

2. The X-ray/computed tomography imaging agent of claim 1, wherein the active nanoparticle core comprises a tantalum oxide

3. The X-ray/computed tomography imaging agent of claim 1 and 2, wherein the active nanoparticle core comprises $Ta_2O_5$.

4. The X-ray/computed tomography imaging agent of claim 1 to 3, wherein the nanoparticle has an average diameter from 1 nm to 20 nm.

5. The X-ray/computed tomography imaging agent of claim 1, wherein the nanoshell comprises material selected from the group consisting of ligands, oligomers, polymers, clusters, carbohydrate species, functionalized silica, and combinations thereof.

6. The X-ray/computed tomography imaging agent of claim 5, wherein the nanoshell comprises material selected from the group consisting of polyethylene glycol, polyethylene imine, polyvinylsulfate, polyvinylpyrrolidinone, citrate, malate, glycolate, silanes, and combinations thereof.

7. The X-ray/computed tomography imaging agent of claim 6, wherein the nanoshell comprises material selected from diethylphosphatoethyltriethoxysilane (PHS) and 2-[Methoxy(poly-ethylenoxy)propyl]trimethoxysilane (PEGsilane550).

8. An X-ray/computed tomography imaging solution, comprising an ensemble of the imaging agents of claim 1 to 7, wherein the mean diameter of the ensemble is not more than 10 nm.

9. A method for making an X-ray/computed tomography imaging agent, the method comprising the steps of:

    a) providing a first precursor material comprising tantalum;
    b) forming an active core from the first precursor material, the core comprising tantalum in a non-zero valent state;
    c) providing a second precursor material; and
    d) forming a passive shell from the second precursor material, wherein the passive shell comprises a polymer selected from the group consisting of polyethylene glycol, polyethylene imine, polyvinylsulfate, and polyvinylpyrrolidinone, and combinations thereof is disposed about the core such that the core and the shell form a core/shell nanoparticle;

    wherein the material of which the nanoshell is made is water soluble and biocompatible.

10. The method of claim 9, wherein the active core comprises $Ta_2O_5$.

11. The method of claim 9 and 10, wherein the second precursor material comprises a polymerizable silane.

12. The method of claim 10, wherein the passive shell comprises a material selected from diethylphosphatoethyltriethoxysilane (PHS) and 2-[Methoxy(polyethylenoxy)propyl]trimethoxysilane (PEGsilane550).

13. The method of claim 9 to 12, wherein the second precursor comprises a carboxylic acid and the passive shell comprises a carboxylic acid selected from the group consisting of citrate, glycolate, and malate.

14. The method of claim 9 to 13, further comprising the step of selecting an average diameter of the nanoparticle by tangential flow filtration, diafiltration or normal filtration.

**Patentansprüche**

1. Röntgenstrahlen/Computertomographiebildgebungsmittel, umfassend :

   a) einen aktiven Nanopartikelkern, wobei der aktive Nanopartikelkern Tantal (Ta) in einem nichtnullwertigen Zustand umfasst;
   b) eine passive Nanoschale, wobei das Material, aus dem die Nanoschale hergestellt ist, wasserlöslich und biokompatibel ist; und
   (c) wobei die Nanoschale derart um den Nanopartikelkern angeordnet ist, dass im Aggregat der aktive Nanopartikelkern und die passive Nanoschale ein Kern-/Schalen-Nanopartikel ausbilden, das zum Gebrauch als Bildgebungsmittel bei der Röntgenstrahlen/Computertomographiebildgebung betriebsfähig ist.

2. Röntgenstrahlen/Computertomographiebildgebungsmittel nach Anspruch 1, wobei der aktive Nanopartikelkern ein Tantaloxid umfasst.

3. Röntgenstrahlen/Computertomographiebildgebungsmittel nach einem der Ansprüche 1 und 2, wobei der aktive Nanopartikelkern $Ta_2O_5$ umfasst.

4. Röntgenstrahlen/Computertomographiebildgebungsmittel nach einem der Ansprüche 1 bis 3, wobei das Nanopartikel einen Durchschnittsdurchmesser von 1 nm bis 20 nm aufweist.

5. Röntgenstrahlen/Computertomographiebildgebungsmittel nach Anspruch 1, wobei die Nanoschale Material umfasst, das aus der Gruppe ausgewählt ist, die aus Liganden, Oligomeren, Polymeren, Clustern, Kohlenhydratarten, funktionalisiertem Siliciumoxid und Kombinationen davon besteht.

6. Röntgenstrahlen/Computertomographiebildgebungsmittel nach Anspruch 5, wobei die Nanoschale Material umfasst, das aus der Gruppe ausgewählt ist, die aus Polyethylenglycol, Polyethylenimin, Polyvinylsulfat, Polyvinylpyrrolidinon, Citrat, Malat, Glycolat, Silanen und Kombinationen davon besteht.

7. Röntgenstrahlen/Computertomographiebildgebungsmittel nach Anspruch 6, wobei die Nanoschale Material umfasst, das aus Diethylphosphatethyltriethoxysilan (PHS) und 2-[Methoxy(polyethylenoxy)propyl]trimethoxysilan (PEGsilan550) ausgewählt ist.

8. Röntgenstrahlen/Computertomographiebildgebungslösung, umfassend ein Ensemble der Bildgebungsmittel von einem der Ansprüche 1 bis 7, wobei der Mitteldurchmesser des Ensembles nicht mehr als 10 nm beträgt.

9. Verfahren zum Herstellen eines Röntgenstrahlen/Computertomographiebildgebungsmittels, wobei das Verfahren die folgenden Schritte umfasst:

   a. Vorsehen eines ersten Vorläufermaterials, das Tantal umfasst;
   b. Ausbilden eines aktiven Kerns aus dem ersten Vorläufermaterial, wobei der Kern Tantal in einem nichtnullwertigen Zustand umfasst;
   c. Vorsehen eines zweiten Vorläufermaterials; und
   d. Ausbilden einer passiven Schale aus dem zweiten Vorläufermaterial, wobei die passive Schale ein Polymer umfasst, das aus der Gruppe ausgewählt ist, die aus Polyethylenglycol, Polyethylenimin, Polyvinylsulfat und Polyvinylpyrrolidinon und Kombinationen davon besteht, und derart um den Kern angeordnet ist, dass der Kern und die Schale ein Kern-/Schalen-Nanopartikel ausbilden;

   wobei das Material, aus dem die Schale hergestellt ist, wasserlöslich und biokompatibel ist.

10. Verfahren nach Anspruch 9, wobei der aktive Kern wobei der aktive Nanopartikelkern $Ta_2O_5$ umfasst.

**11.** Verfahren nach einem der Ansprüche 9 und 10, wobei das zweite Vorläufermaterial ein polymerisierbares Silan umfasst.

**12.** Verfahren nach Anspruch 10, wobei die passive Schale Material umfasst, das aus Diethylphosphatethyltriethoxysilan (PHS) und 2-[Methoxy(polyethylenoxy)propyl]trimethoxysilan (PEGsilan550) ausgewählt ist.

**13.** Verfahren nach einem der Ansprüche 9 bis 12, wobei der zweite Vorläufer eine Karbonsäure umfasst und die passive Schale eine Karbonsäure umfasst, die aus der Gruppe ausgewählt ist, die aus Citrat, Glycolat und Malat besteht.

**14.** Verfahren nach einem der Ansprüche 9 bis 13, ferner umfassend den Schritt des Auswählens eines Durchschnittsdurchmessers des Nanopartikels durch Tangentialflussfiltration, Diafiltration oder normale Filtration.

**Revendications**

**1.** Agent d'imagerie pour tomographie aux rayons X assistée par ordinateur comprenant :

a) un noyau nanoparticulaire actif, dans lequel le noyau nanoparticulaire actif comprend du tantale (Ta) dans un état de valence non nulle ;
b) une nanocoque passive, dans lequel le matériau dont la nanocoque est constituée est soluble dans l'eau et biocompatible et
(c) la nanocoque est disposée autour du noyau nanoparticulaire de sorte que, dans l'agrégat, le noyau nanoparticulaire actif et la nanocoque passive forment une nanoparticule de type noyau/coque qui peut être utilisée comme agent d'imagerie en imagerie pour tomographie aux rayons X assistée par ordinateur.

**2.** Agent d'imagerie pour tomographie aux rayons X assistée par ordinateur selon la revendication 1, dans lequel le noyau nanoparticulaire actif comprend un oxyde de tantale.

**3.** Agent d'imagerie pour tomographie aux rayons X assistée par ordinateur selon les revendications 1 et 2, dans lequel le noyau nanoparticulaire actif comprend du $Ta_2O_5$.

**4.** Agent d'imagerie pour tomographie aux rayons X assistée par ordinateur selon les revendications 1 et 3, dans lequel la nanoparticule a un diamètre moyen de 1 nm à 20 nm.

**5.** Agent d'imagerie pour tomographie aux rayons X assistée par ordinateur selon la revendication 1, dans lequel la nanocoque comprend un matériau choisi dans le groupe constitué des ligands, des oligomères, des polymères, des amas, de types d'hydrates de carbone, de la silice fonctionnalisée et de leurs combinaisons.

**6.** Agent d'imagerie pour tomographie aux rayons X assistée par ordinateur selon la revendication 5, dans lequel la nanocoque comprend un matériau choisi dans le groupe constitué du polyéthylèneglycol, d'une polyéthylèneimine, d'un poly(sulfate de vinyle), de polyvinylpyrrolidinone, d'un citrate, d'un malate, d'un glycolate, de silanes et de leurs combinaisons.

**7.** Agent d'imagerie pour tomographie aux rayons X assistée par ordinateur selon la revendication 6, dans lequel la nanocoque comprend un matériau choisi parmi le diéthylphosphatoéthyltriéthoxysilane (PHS) et le 2-[méthoxy(polyéthylèneoxy)-propyl]triméthoxysilane (PEGsilane550).

**8.** Solution d'imagerie pour tomographie aux rayons X assistée par ordinateur comprenant un ensemble des agents d'imagerie des revendications 1 à 7, dans laquelle le diamètre moyen de l'ensemble n'est pas supérieur à 10 nm.

**9.** Procédé de fabrication d'un agent d'imagerie pour tomographie aux rayons X assistée par ordinateur, le procédé comprenant les étapes consistant à :

a) fournir un premier matériau précurseur comprenant du tantale ;
b) former un noyau actif à partir du premier matériau précurseur, le noyau comprenant du tantale dans un état de valence non nulle ;
c) fournir un second matériau précurseur ; et
d) former une coque passive à partir du second matériau précurseur, dans lequel la coque passive comprend un polymère choisi dans le groupe constitué du polyéthylèneglycol, d'une polyéthylèneimine, d'un poly(sulfate de vinyle) et de polyvinylpyrrolidinone ainsi que de leurs combinaisons, qui est disposé autour du noyau de sorte que le noyau et la coque forment une nanoparticule de type noyau/coque;

dans lequel le matériau dont la nanocoque est constituée est soluble dans l'eau et biocompatible.

**10.** Procédé selon la revendication 9, dans lequel le noyau actif comprend du $Ta_2O_5$.

**11.** Procédé selon les revendications 9 et 10, dans lequel le second matériau précurseur comprend un silane polymérisable.

**12.** Procédé selon la revendication 10, dans lequel la coque passive comprend un matériau choisi parmi le diéthylphosphatoéthyltriéthoxysilane (PHS) et le 2-[méthoxy(polyéthylèneoxy)propyl]triméthoxysilane (PEGsilane550).

**13.** Procédé selon les revendications 9 à 12, dans lequel le second précurseur comprend un acide carboxylique et la coque passive comprend un acide carboxylique choisi dans le groupe constitué d'un citrate, d'un glycolate et d'un malate.

**14.** Procédé selon les revendication 9 à 13, comprenant en outre l'étape de sélection d'un diamètre moyen de la nanoparticule par filtration à écoulement tangentiel, diafiltration ou filtration normale.

## FIG. 1

100 — 102

101

## FIG. 2

200 — 202

201

## FIG. 3

| | |
|---|---|
| PROVIDING A QUANTITY OF CORE/SHELL NONAPARTICLES OPERABLE FOR USE AS CT IMAGING AGENTS | ─STEP 301 |

↓

| | |
|---|---|
| ADMINISTERING THE CORE/SHELL NANOPARTICLES TO A MAMMALIAN SUBJECT | ─STEP 302 |

↓

| | |
|---|---|
| IRRADIATING THE SUBJECT WITH X-RAYS IN ACCORDANCE WITH COMPUTED TOMOGRAPHY AS AN IMAGING AGENT | ─STEP 303 |

*FIG. 4*

*FIG. 5*

*FIG. 6*

## FIG. 7

20 nm

## FIG. 8

◇ Ta PEG550 (24% Ta)
□ Ta CITRATE
○ Ta PEG550 (35% Ta)

% VIABLE CELLS

[Ta] M

## FIG. 9

A450

C3a (ng/mL)

## FIG. 10

## FIG. 11

## FIG. 12

PREINJECTION

6 MINUTES POST

14 MINUTES POST

23 MINUTES POST

## REFERENCES CITED IN THE DESCRIPTION

### Patent documents cited in the description

- WO 03075961A2 A
- WO 2005051435 A2
- WO 03075961 A2
- WO 07055995 A2, Bonitatebus
- WO 03016217 A

### Non-patent literature cited in the description

- **SHI-BAO YU ; ALAN D. WATSON.** *Chem. Rev.,* 1999, vol. 99, 2353-2377
- **LEIKE et al.** *Invest. Radiol.,* 2001, vol. 36 (6), 303-308
- **YORDANOV et al.** *Nano Letters,* 2002, vol. 2 (6), 595-599
- **CHAN et al.** *Dental Materials,* 1999, vol. 15 (3), 219-222
- **BRAUNE et al.** *Proc. SPIE 3469, Organic-Inorganic Hybrid Materials for Photonics,* 1998, 124-132
- **IWASAKI et al.** *J Phys Chem,* 2004, vol. 108 (32), 11946-11952
- **WANG et al.** *Colloids and Surfaces, B: Biointerfaces,* 2005, vol. 46 (4), 255-260
- **KANNAN et al.** *J Vacuum Sci Technol B: Microelectronics and Nanometer Structures - Processing, Measurement and Phenomena,* 2005, vol. 23 (4), 1364-1370
- **PAPRA et al.** *Langmuir Am Chem Soc USA,* 2001, vol. 17 (5), 1457-1460
- Handbook of Medical Imaging, Volume 1, Physics and Psychophysics. SPIE Press, 2000, vol. 1
- **G. T. HERMANSON.** Bioconjugate Techniques. Academic Press, 1996
- **SWEENEY et al.** *J. Am. Chem. Soc.,* 2006, vol. 128, 3190-3197